# EUROPEAN PATENT APPLICATION

(11) **EP 4 410 984 A1**
(43) Date of publication of application: **07.08.2024**
(21) Application number: 22874472.8
(22) Date of filing: 19.08.2022
(51) Int. Cl.: C12N 15/74, C12N 1/21, C12N 15/54, C12N 15/53, C12N 15/52, C12P 7/62, C12R 1/01

(54) **GENETICALLY ENGINEERED STRAIN FOR PRODUCING POLYLACTIC ACID AND METHOD FOR PRODUCING POLYLACTIC ACID**

(30) Priority: 29.09.2021 CN 202111147519
(71) Applicant: Shanghai Sipeng Technology Ltd., Shanghai 201111 (CN)
(72) Inventor: TAO, Fei, Shanghai 200240 (CN); TAN, Chunlin, Shanghai 200240 (CN); XU, Ping, Shanghai 200240 (CN)
(74) Representative: Prinz & Partner mbB
(86) International application number: PCT/CN2022/113485
(87) International publication number: WO 2023/051077

(57) **Abstract**

Provided are a genetically engineered strain for producing polylactic acid and a method for producing polylactic acid. The method includes: synthesizing target gene using D-lactate dehydrogenase gene, propionyl-CoA transferase gene, and polyhydroxyalkanoate synthase gene; and performing double enzymatic cleavage on a vector; purifying; and transforming competent cells to obtain a recombinant plasmid; extracting DNA of the recombinant plasmid; introducing the DNA into an engineering strain, and extracting and verifying to obtain an engineering cell strain; and then culturing the engineering cell strain under light in a culture medium, in which carbon dioxide is introduced; centrifuging; drying; and recycling. According to the present application, by modifying natural cyanobacteria and using a high density fermentation strategy, the biomass of the cyanobacteria is significantly increased. According to the present application, the problem in the related art, i.e., polylactic acid can be only produced by using sugar-derived raw material, is solved.

## Description

### FIELD

The present disclosure belongs to the technical field of bioengineering, and particularly, relates to a genetically engineered strain for producing polylactic acid (PLA) and a method for producing PLA.

### BACKGROUND

PLA is a promising biodegradable material with broad application prospects in disposable packaging, agriculture, medicine, 3D printing and other fields. Currently, PLA is mainly produced by producing lactate through microbial fermentation, converting lactate into lactide and then cyclizing the lactide ring. However, such chemical polymerization has high requirements for equipment, and the required solvents or chain coupling agents can be hardly removed, which greatly limits the development of commercial production of PLA. Therefore, it is of great significance to develop a method for producing high-performance PLA. Currently, the biological production of PLA relies mainly on a sugar-derived raw material, which may also directly result in competition between industrial production and food, thereby causing potential risks. At the same time, with the advancement in industrialization, the emission of greenhouse gas CO₂ has been dramatically increased, resulting in serious global climate problems.

### SUMMARY

Regarding the deficiencies in the related art, objectives of the present disclosure is to provide a genetically engineered strain for producing PLA and a method for producing PLA.

As for the above obj ectives, the present disclosure provideds the following solutions.
(1) A genetically engineered strain of *Synechococcus elongatus,* characterized in that, the genome of the genetically engineered strain is integrated with a coding sequence of exogenous D-lactate dehydrogenase gene, a coding sequence of exogenous propionyl-CoA transferase gene, and a coding sequence of exogenous polyhydroxyalkanoate synthase gene, enabling the genetically engineered strain to express exogenous D-lactate dehydrogenase, exogenous propionyl-CoA transferase, and exogenous polyhydroxyalkanoate synthase.
(2) The genetically engineered strain according to item (1), wherein the coding sequence of D-lactate dehydrogenase gene is under the control of a P_{trc} promoter, a P_{psba} promoter or a P_{cpc560} promoter.
(3) The genetically engineered strain according to item (1), wherein the coding sequence of propionyl-CoA transferase gene is under the control of a P_{trc} promoter, a P_{psba} promoter, or a P_{cpc560} promoter.
(4) The genetically engineered strain according to item (1), wherein the coding sequence of polyhydroxyalkanoate synthase gene is under the control of a P_{trc} promoter, a P_{psba} promoter, or a P_{cpc560} promoter.
(5) The genetically engineered strain according to item (1), wherein the coding sequence of exogenous D-lactate dehydrogenase gene is derived from *Lactobacillus bulgaricus;* the coding sequence of exogenous propionyl-CoA transferase gene is derived from *Clostridium propionicum;* and the coding sequence of exogenous polyhydroxyalkanoate synthase gene is derived from *Pseudomonas.*
(6) The genetically engineered strain according to item (1), whereinthe genome of the genetically engineered strain is integrated with a coding sequence of exogenous acetyl-CoA synthase gene, enabling the genetically engineered strain to express the exogenous acetyl-CoA synthase gene.
(7) The genetically engineered strain according to item (1), wherein expression of acetate kinase gene in the genetically engineered strain is knocked down.
(8) The genetically engineered strain according to item (7), wherein the genome of the genetically engineered strain is integrated with a coding sequence of exogenous sRNA capable of inhibiting the expression of acetate kinase gene.
(9) The genetically engineered strain according to (1), wherein expression of acetyl-CoA carboxylase gene, expression of ketosynthase gene, and/or expression of beta-ketoacyl-ACP synthase III gene in the genetically engineered strain are knocked down.
(10) The genetically engineered strain according to item (9), wherein the genome of the genetically engineered strain is integrated with a coding sequence of exogenous sRNA capable of inhibiting the expression of acetyl-CoA carboxylase gene, the expression of ketosynthase gene, and/or the expression of beta-ketoacyl-ACP synthase III gene.
(11) The genetically engineered strain according to any one of items (1) to (10), wherein the genetically engineered strain is a *Synechococcus elongatus* PCC7942 strain.
(12) A method for producing polylactic acid using carbon dioxide, including:
   1) providing the genetically engineered strain of *Synechococcus elongatus* according to any one of items (1) to (11);
   2) introducing carbon dioxide and culturing the genetically engineered strain under light; and
   3) when a growth OD of the genetically engineered strain reaches the maximum, collecting and drying the fermented cells, and recycling the polylactic acid in the cells.
(13) The method according to item (12), wherein in step 2), the carbon dioxide is introduced with a ventilation rate of 1.5 vvm, and a volume ratio of introduced carbon dioxide to air is 1: (1 to 10).
(14) The method according to item (12), wherein in step 2), an intensity of the light is from 125 µmol photons m⁻² s⁻¹ to less than 1000 µmol photons m⁻² s⁻¹, and preferably, the intensity of the light ranges from 125 µmol photons m⁻² s⁻¹ to 500 µmol photons m⁻² s⁻¹.
(15) The method according to item (12), wherein in step 2), the genetically engineered strain is cultured in a culture medium including sodium carbonate, sodium nitrate, dipotassium hydrogen phosphate, magnesium sulfate, calcium chloride, citric acid, ferric ammonium citrate, ethylenediamine tetraacetic acid, boric acid, manganese chloride, zinc sulfate, sodium molybdate, copper sulfate, and cobalt nitrate.
(16) The method according to item (15), wherein Na₂CO₃, MgSO₄·7H₂O, NaNO₃, KH₂PO₄, and trace elements are further added to the formula of the culture medium, and preferably, the trace elements are selected from H₃BO₃, MnCl₂7H₂O, ZnSO₄·7H₂O, Na₂MoO₄·2H₂O, CuSO₄ ·5H₂O, and Co(NO₃)₂·6H₂O.
(17) The method according to item (15) or item (16), wherein a final concentration of the sodium carbonate in the culture medium ranges from 0.25 g/L to 1.5 g/L.
(18) The method according to item (12), wherein in step 2), the genetically engineered strain is cultured in the presence of an inducer, and preferably, the inducer is selected from one or more of IPTG and theophylline.
(19) The method according to item (12), wherein in step 3), said collecting the genetically engineered strain includes: mixing a polyelectrolyte flocculant with a culture solution containing the genetically engineered strain for 5 minutes, standing for 5 minutes, discarding a supernatant, and collecting a precipitate. Preferably, the polyelectrolyte flocculant is a polyacrylamide flocculant.
(20) The method according to item (12), wherein in step 3), said recycling the polylactic acid in the strain is performed by using an organic solvent recycling or an inorganic aqueous phase extraction method. Preferably, the organic solvent is selected from chloroform, 1,4-epoxydioxane, methyl-tetrahydrofuran, ethylene carbonate, acetone, and ethyl acetate.

With the above solutions, the present disclosure provides the following beneficial effects.

According to the present disclosure, natural *Synechococcus elongatus* (also referred to herein as "cyanobacteria") is used as a starting strain, and heterologous D-lactate dehydrogenase (LDH), propionyl-CoA transferase (PCT), and polyhydroxyalkanoate synthase (PHA) are introduced by means of the genetic engineering technology, thereby successfully synthesizing PLA through the metabolic pathway of *Synechococcus elongatus.* In addition, according to the present disclosure, different metabolic engineering strategies, including promoter optimization for rate-limiting enzymes, construction of acetyl CoA self-cycling, and carbon flux redirection, are systematically employed. That is, the promoters of rate-limiting enzymes, i.e., PCT and PHA, are optimized to overexpress acetyl-CoA synthase and weaken the expression of acetate kinase, and the genes of key enzymes of a fatty acid pathway such as acetyl-CoA carboxylase, ketosynthase and beta-ketoacyl-ACP synthase III are knocked down. In this way, the yield of PLA is further enhanced.

Meanwhile, according to the present disclosure, a high density culture strategy (HDC), including the conditions for introducing CO₂ and light into the fermentation process, is developed. The high density fermentation strategy enables easy adjustment of high and low light and combined use of different types of light (a combination of red and white light). In addition, according to the present disclosure, it is also possible to automatically introduce and stirr during condensation and fermentation, with a stirring speed of 200 rpm under culture conditions. According to the present disclosure, the yield of PLA is further enhanced by exploring the conditions for culture and fermentation.

The present disclosure discloses the production of plastics from carbon dioxide using a cyanobacteria cell factory.

According to the present disclosure, by modifying natural cyanobacteria (*Synechococcus elongatus*) and using a high density fermentation strategy, CO₂ and light can be directly used to produce PLA, and the biomass of the cyanobacteria is significantly increased. According to the present disclosure, the problem in the related art, i.e., polylactic acid can be only produced by using sugar-derived raw material, is solved, and production costs and collection costs are reduced. In the meantime, it is also promising to solve the problems of plastic pollution and excessive emission of greenhouse gas CO₂, while biodegradable material PLA can be produced, thereby having great potential industrial application value and accelerating the development of commercial production of PLA with cyanobacteria. In addition, according to the present disclosure, the cyanobacteria cells can be very simply and conveniently collected by using an ampholyte polyacrylamide flocculant.

### BRIEF DESCRIPTION OF THE DRAWINGS

Fig. 1 is a schematic diagram of a synthetic route and metabolic engineering strategy for PLA according to an embodiment of the present disclosure.
Fig. 2 illustrates a hydrogen spectrum and a carbon spectrum for nuclear magnetic detection of PLA synthesized in Example 7 of the present disclosure. Fig. 2A illustrates a hydrogen spectrum; Fig. 2B illustrates a carbon spectrum; Fig. 2C illustrates a structural formula of PLA; and Fig. 2D illustrates a ¹H-¹H two-dimensional COSY diagram.

### DETAILED DESCRIPTION

According to an aspect of the present disclosure, a genetically engineered strain of *Synechococcus elongatus* is provided. The genome of the genetically engineered strain is integrated with a coding sequence of exogenous D-lactate dehydrogenase gene, a coding sequence of exogenous propionyl-CoA transferase gene, and a coding sequence of exogenous polyhydroxyalkanoate synthase gene, enabling the genetically engineered strain to express exogenous D-lactate dehydrogenase, exogenous propionyl-CoA transferase, and exogenous polyhydroxyalkanoate synthase.

In some embodiments, the coding sequence of exogenous D-lactate dehydrogenase gene is derived from *Lactobacillus bulgaricus,* the coding sequence of exogenous propionyl-CoA transferase gene is derived from *Clostridium propionicum,* and the coding sequence of exogenous polyhydroxyalkanoate synthase gene is derived from *Pseudomonas.*

In the present disclosure, the coding sequence of D-lactate dehydrogenase gene, the coding sequence of propionyl-CoA transferase gene, and the coding sequence of polyhydroxyalkanoate synthase gene may be wild-type or may be appropriately modified. According to the present disclosure, it is found that the yield of PLA can be improved when the gene coding sequences of the above-mentioned three enzymes are mutated in accordance with the diclsoure of *"*Yang et al., Biotechnology & Bioengineering, 2010, 105 (1): 150-160*." and "*Li, C., Tao, F., Ni, J. et al. Enhancing the light-driven production of D-lactate by engineering cyanobacterium using a combinational strategy. Sci Rep 5, 9777 (2015*)",* entire contents of which is incorporated herein by reference.

The term "exogenous" as used herein in reference to a gene, a coding sequence, a protein, an enzyme, etc., refers to a substance that does not naturally belong to a specific strain. For example, an exogenous gene refers to a gene introduced into a specific strain from outside. The exogenous gene may be a gene existing in the genome of the strain or a gene not existing in the genome. In an embodiment of the present disclosure, an existing acetyl-CoA synthase gene is introduced into the genome of a *Synechococcus elongatus* strain from outside to overexpress this gene as needed.

According to the present disclosure, the promoter for controlling the transcription of the exogenous gene is further optimized, resulting in a further improvement in the yield of PLA. Preferably, the coding sequence of D-lactate dehydrogenase gene is under the control of a P_{trc} promoter, a P_{psba} promoter, or a P_{cpc560} promoter. Further preferably, the coding sequence of propionyl-CoA transferase gene is under the control of a P_{trc} promoter, a P_{psba} promoter, or a P_{cpc560} promoter. Further preferably, the coding sequence of polyhydroxyalkanoate synthase gene is under the control of a P_{trc} promoter, a P_{psba} promoter, or a P_{cpc560} promoter.

According to the present disclosure, it is found that the genetically engineered strain can express the exogenous acetyl-CoA synthase gene by further integrating the genome of the genetically engineered strain with the coding sequence of exogenous acetyl-CoA synthase gene, thereby resulting in a further improvement in the yield of PLA.

According to the present disclosure, it is also found that the yield of PLA can be further improved by weakening the expression of acetate kinase and/or knocking down the genes of key enzymes of a fatty acid pathway, including genes of acetyl-CoA carboxylase, ketosynthase and beta-ketoacyl-ACP synthase III.

Therefore, it is preferred that the expression of the acetate kinase gene in the genetically engineered strain is knocked down, which can be achieved by integrating the genome of the strain with a coding sequence of exogenous sRNA capable of inhibiting the expression of the acetate kinase gene.

It is further preferred that, the expression of acetyl-CoA carboxylase gene, the expression of ketosynthase gene, and/or the expression of beta-ketoacyl-ACP synthase III gene in the genetically engineered strain are knocked down, which may be achieved by integrating the genome of the strain with a coding sequence of exogenous sRNA capable of inhibiting the expression of acetyl-CoA carboxylase gene, the expression of ketosynthase gene, and/or the expression of beta-ketoacyl-ACP synthase III gene.

In some specific embodiments, the genetically engineered strain is a *Synechococcus elongatus* PCC 7942 strain.

According to another aspect of the present disclosure, a method for producing polylactic acid using carbon dioxide is provided. The method inclures the following steps:
1) providing the genetically engineered strain of *Synechococcus elongatus* according to any one of claims 1-11;
2) introducing carbon dioxide and culturing the genetically engineered strain under light; and
3) when a growth OD of the genetically engineered strain reaches the maximum, collecting and drying the genetically engineered strain, and recycling the polylactic acid in the strain.

Preferably, in step 2), the carbon dioxide is introduced with a ventilation rate of 1.5 vvm. Further preferably, a volume ratio of introduced carbon dioxide to air is 1: (1 to 10), and most preferably, 5%.

Preferably, in step 2), an intensity of the light is from 125 µmol photons m⁻² s⁻¹ to less than 1000 µmol photons m⁻² s⁻¹; more preferably, the intensity of the light ranges from 125 µmol photons m⁻² s⁻¹ to 500 µmol photons m⁻² s⁻¹, and most preferably, 500 µmol photons m⁻² s⁻¹.

Further, according to the present disclosure, it is found that the genetically engineered strain should not be subjected to excessive light at the initial stage of growth. At this time, the light intensity of 150 µmol photons m⁻² s⁻¹ or less is preferareble, and the light intensity of 125 µmol photons m⁻² s⁻¹ is most preferareble. With the growth of the strain, the light intensity is gradually increased to 500 µmol photos m⁻² s⁻¹, enabling the strain to grow best.

According to the present disclosure, a culture medium for culturing the strain is also developed. It is found that the culture medium having the following formula can increase the biomass of the genetically engineered strain. The culture medium includes sodium carbonate, sodium nitrate, dipotassium hydrogen phosphate, magnesium sulfate, calcium chloride, citric acid, ferric ammonium citrate, ethylenediamine tetraacetic acid, boric acid, manganese chloride, zinc sulfate, sodium molybdate, copper sulfate, and cobalt nitrate. More preferably, Na₂CO₃, MgS0O₄·7H₂O, NaNO₃, KH₂PO₄, and trace elements are further added to the above formula. Preferably, the trace elements are selected from H₃BO₃, MnCl₂7H₂O, ZnSO₄·7H₂O, Na₂MoO₄·2H₂O, CuSO₄·5H₂O, and Co(NO₃)₂·6H₂O.

According to the present disclosure, it is found that a final concentration of the sodium carbonate in the culture medium ranges from 0.25 g/L to 1.5 g/L.

Preferably, in step 2), the genetically engineered strain is cultured in the presence of an inducer to further enhance the yield of PLA. The inducer is preferably selected from one or more of IPTG and theophylline.

In step 3), said collecting the genetically engineered strain can be performed by: mixing a polyelectrolyte flocculant with a culture solution containing the genetically engineered strain for 5 minutes, standing for 5 minutes, discarding a supernatant, and collecting a precipitate. Preferably, the polyelectrolyte flocculant is a polyacrylamide flocculant.

Said drying the genetically engineered strain may be performed by using freeze-drying.

In step 3), said recycling polylactic acid in the strain is performed by using an organic solvent recycling or an inorganic aqueous phase extraction method.

The organic solvent used can be selected from chloroform, 1,4-epoxydioxane, methyl-tetrahydrofuran, ethylene carbonate, acetone, and ethyl acetate.

The inorganic aqueous phase extraction method may include the following steps: (a) providing dried bacterial cells containing polymer-rich particles; (b) pulverizing the dried cell particles; (c) dissolving the pulverized cell powder obtained in step (b) with an alkaline aqueous solution (pH 8 to 10); (d) homogenizing the polymer-rich alkaline solution; (e) separating fragments of non-target polymers remaining within the cells to obtain a first product; and (f) filtering and drying the enriched substances to obtain polymer particles.

A technical and economic bottleneck of the algal biomass industry is to harvest microalgae biomass on an industrial scale. The situation could be more complex when it comes microalgae due to the small cell size and the biomass concentration diluted in the culture. During the harvest, a huge amount of water is required to be removed, making such a process to be energy and cost intensive, which may account for approximately 30% of the total cost of biomass production. PLA is an inclusion of cells, and thus PLA particles can be collected together with the cells by centrifugation. According to the present disclosure, it is found that the cells can be collected in a more quick and easier way when using a flocculant, thereby having great potential industrial value.

In a specific embodiment of the present disclosure, the method for producing polylactic acid using carbon dioxide includes the following steps:
(1) synthesizing modified D-lactate dehydrogenase gene, modified propionyl-CoA transferase gene, and modified polyhydroxyalkanoate synthase gene into a target gene using primers, and performing double enzymatic cleavage on the target gene and a vector, purifying, and transforming the vector into *E. coli* competent cells to obtain a recombinant plasmid;
(2) extracting DNA of the recombinant plasmid and introducing the DNA into cyanobacteria, i.e., *S. elongatus* PCC 7942, followed by extracting and verifying, to obtain an engineering cell strain; and
(3) culturing the engineering cell strain in a culture medium, introducing carbon dioxide and light into the culture medium, followed by centrifuging, collecting, freeze-drying, and recycling, to obtain PLA.

According to the present disclosure, a high density culture strategy (HDC), including the conditions for introducing CO₂ and light into the culture medium, is developed. The high density fermentation strategy enables easy adjustment of high and low light and combined use of different types of light (a combination of red and white light). In addition, according to the present disclosure, it is also possible to automatically introduce and stirr during condensation and fermentation, with a stirring speed of 200 rpm under culture conditions. According to the present disclosure, the yield of PLA is further enhanced by exploring the conditions for culture and fermentation.

### EXAMPLES

The technical contents of the present disclosure will be further described with reference to the following examples. The following examples are illustrative, rather than restrictive, and they are not intended to limit the scope of the present disclosure. The experimental methods used in the following examples are conventional methods, unless otherwise specified. The materials, reagents, etc. used in the following examples are all commercially available, unless otherwise specified.

BG-11 liquid culture medium (1X): 1.5 g of NaNO₃, 0.04 g of K₂HPO₄·3H₂O, 0.075 g of MgSO₄·7H₂O, 0.036 g of CaCl₂·2H₂O, 0.006 g of citric acid, 0.006 g of ferric ammonium citrate, 0.001 g of EDTA, 0.02 g of Na₂CO₃, and 1 mL of metal ion mother liquor (2.86 g/L of boric acid, 1.81 g/L of MnCl₂·4H₂O, 0.222 g/L of ZnSO4·7H₂O, 0.39 g/L of Na₂MoO₄·2H₂O, 0.079 g/L of CuSO₄·5H₂O, and 0.0494 g/L of Co(NO₃)₂·6H₂O) were dissolved and made up in 1 L of distilled water. Autoclave sterilization was performed at 121°C for 20 min.

BG-11 solid culture medium: 1.6 to 2.0% (w/v) agar powder was added to the BG-11 liquid culture medium. Autoclave sterilization was performed at 121°C for 20 min.

Strain culture method for PLA production: S. *elongatus* PCC 7942 cells in exponential phase were taken and diluted in a 300 mL conical flask containing 100 mL of BG-11 liquid culture medium to the final concentration of the cells of OD₇₃₀ₙₘ = 0.05. The culture medium was supplemented with 20 mg/L of spectinomycin. When the strain grew to OD₇₃₀ₙₘ=0.4-0.6, 1 mmol/L isopropyl-beta-D-thiogalactoside (IPTG) was added for induction. 2 mL of sample was taken daily for cell growth determination. After each sampling, an equal volume of sterilized BG-11 liquid culture medium was supplemented to the conical flask.

### Example 1: Introduction of metabolic pathway for PLA synthesis

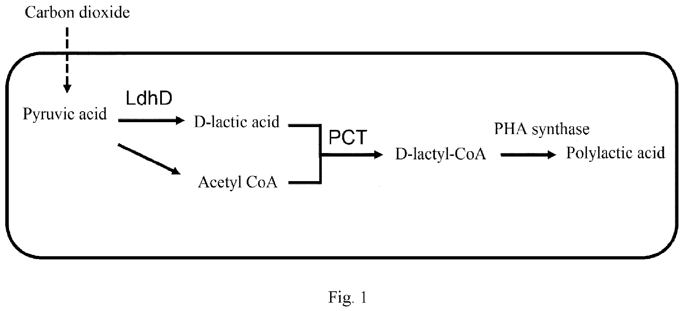

As illustrated in Fig. 1, based on the reported coding sequences of engineered D-lactate dehydrogenase gene (*ldhD*), propionyl-CoA transferase gene *(pct),* and polyhydroxyalkanoate synthase gene (*pha*) (see, *"*Yang et al. Biotechnology & Bioengineering, 2010, 105 (1): 150-160*." and "*Li, C. Tao, F. Ni, J. et al. Enhancing the light-driven production of D-lactate by engineering cyanobacterium using a combinational strategy. Sci Rep 5, 9777 (2015*)"),* a full-length sequence containing the engineered gene coding sequences of the above three enzymes was artificially synthesized in the order of *IdhD, pct* and *pha* with RBS sequence (SEQ ID NO. 19: AAGAAGGAGATATACC) inserted between the two genes. The synthesized sequence, as a template, was amplified by PCR using the following primers to obtain a recombinant gene fragment containing the three key enzymes.
SEQ ID NO. 1: 5-ACTCGAGATGAGCAACAAGTCAAATGATGAA-3;
SEQ ID NO. 2: 5-GGATCCTCTAGGACTTCATTTCTTTCAGGCC-3.

The recombinant gene and pAMMCS12 were double enzymatically cleaved with *Xho*I and *BamH*I*,* respectively, purified, and then ligated overnight at 16°C using T₄ DNA ligase. The ligation product was chemically transformed into *E. coli* competent cells. The transformed mixture was spread on an LB plate with spectinomycin (50 µg/mL). The plasmid was extracted. Double enzymatic cleavage was performed to verify the constructed recombinant plasmid named pAM*-ldhD-pct-pha.* Sequencing was performed by Shanghai Parsenor Biotechnology Co., Ltd.

The target gene was integrated into the chromosome of *Synechococcus elongatus* PCC 7942 (purchased from the Global Center for Biological Resources, ATCC) via the extracted recombinant plasmid DNA by natural transformation using the principle of homologous recombination. The genome of the recombinant transformants was extracted and amplified by PCR using verification primers, SEQ ID NO. 3: 5-ACCTGGAATTGGTTGAAGG-3 and SEQ ID NO. 4: 5-ACAGCCAAGCTTGCATGC-3, to verify the successful insertion to obtain a PYW01 strain. According to the method described in Example 7, the yield of the obtained PYLW01 strain was determined to be 0.8 mg g⁻¹ DCW.

### Example 2: Optimization for promoters of rate-limiting enzymes

Based on a P_{trc} promoter, the gene synthesis was performed, and primers were designed as follows: SEQ ID NO. 5: 5-CGACTGCACGGTGCACCA-3; SEQ ID NO. 6: 5-CATGGTCTGTTTCCTGTGTGAA-3. The P_{trc} promoter was obtained by PCR amplification.

Based on a P_{psba} promoter, the gene synthesis was performed, and primers were designed as follows: SEQ ID NO. 7: 5- TATCAATAAGTATTAGGTATATGG-3; SEQ ID NO. 8: 5- ATGTATTTGTCGATGTTCAGAT-3. The P_{psba} promoter was obtained by PCR amplification.

Based on a P_{cpc560} promoter, the gene synthesis was performed, and primers were designed as follows: SEQ ID NO. 9: 5- ACCTGTAGAGAAGAGTCCCT-3; SEQ ID NO. 10: 5-TGAATTAATCTCCTACTTGACTTTA-3. The P_{cpc560} promoter was obtained by PCR amplification.

1 µL of the P_{trc} promoter sequence obtained by PCR amplification, and 1 µL of the above three gene coding sequences, *IdhD, pct* and *pha,* were taken, respectively. First, a P_{trc}-*ldhD* fragment, a P_{trc}-*pct* fragment, and a P_{trc}-*pha* fragment were generated by means of overlap extension PCR, respectively. Then, these three fragments were successively ligated to a pAM vector by *EcoR*I*, BamH*I*,* and *XhoI* enzymatic cleavage to obtain a vector pAM-P_{trc}-*ldhD*-P_{trc}-*pct*-P_{trc}-*pha.*

1 µL of the P_{psba} promoter sequence obtained by PCR amplification, and 1 µL of the above three gene coding sequences, *IdhD, pct* and *pha*, were taken, respectively. First, a P_{psba}-*IdhD* fragment, a P_{psba}-*pct* fragment, and a P_{psba}-*pha* fragment were generated by means of overlap extension PCR, respectively. Then, these three fragments were successively ligated into a pAM vector by *EcoRI, BamHI,* and *XhoI* enzymatic cleavage to obtain a vector pAM-P_{psba}-*IdhD- P*_{psba}*-pct- P*_{psba}*-pha.*

1 µL of the P_{cpc560} promoter sequence obtained by PCR amplification, and1 µL of the above three gene coding sequences, *IdhD, pct* and *pha,* were taken, respectively. First, a P_{cpc560}-*ldhD* fragment, a P_{cpc560}-*pct* fragment, and a P_{cpc560}-*pha* fragment were generated by means of overlap extension PCR, respectively. Then, these three fragments were successively ligated into a pAM vector by *EcoRI, BamHI,* and *XhoI* enzymatic cleavage to obtain a vector pAM-P_{cpc560}-*ldhD*-P_{cpc560}-*pct*-P_{cpc560}-*pha.*

### Example 3: Construction of PYLW02, PYLW03 and PYLW004 engineering strains

The vector pAM-P_{trc}-*ldhD*-P_{trc}-*pct*-P_{trc}-*pha* was integrated into the neutral site I in the chromosome of *Synechococcus elongatus* PCC 7942 by natural transformation to obtain a PYLW02 strain.

The vector pAM-P_{psba}-*ldhD*-P_{psba}-*pct*-P_{psba}-*pha* was integrated into the neutral site I in the chromosome of *Synechococcus elongatus* PCC 7942 by natural transformation to obtain a PYLW03 strain.

The vector pAM-P_{cpc560}-*ldhD*-P_{cpc560}-*pct*-P_{cpc560}-*pha* was integrated into the neutral site I in the chromosome of *Synechococcus elongatus* PCC 7942 by natural transformation to obtain a PYLW04 strain.

According to the method described in Example 7, the yield of the PYLW02 strain was the highest, determined to be 5.6 mg g⁻¹DCW.

### Example 4: Overexpression of acetyl-CoA synthase

Based on the genome (NC_007595.1) sequence of *Synechococcus elongatus* PCC 7942, primers were designed as follows:
SEQ ID NO. 11: 5-ACTAGTATGGCTGCACCTGTCACGAA-3;
SEQ ID NO. 12: 5-GGATCCTTAGTCACTGCTTTCCAGAAACAC-3.

The recombinant gene *acsA* (GenBank: ABB57382.1) and pBA3031M were double enzymatically cleaved with *SpeI* and *BamHI,* respectively, purified, and then ligated overnight at 16°C using T₄ DNA ligase. The ligation product was chemically transformed into *E. coli* competent cells. The transformed mixture was spread on an LB plate with kanamycin (50 µg/mL). The plasmid was extracted. Double enzymatic cleavage was performed to verify the constructed recombinant plasmid named *pBA-acsA.* Sequencing was performed by Shanghai Parsenor Biotechnology Co., Ltd.

The target gene was integrated into the natural site I in the chromosome of *Synechococcus elongatus* PCC 7942 via the extracted recombinant plasmid DNA by natural transformation based on the principle of homologous recombination. The genome of the recombinant transformants was extracted and amplified by PCR using verification primers, i.e., SEQ ID NO. 13: 5-AGGGCTTCCCGGTATCAA-3 and SEQ ID NO. 14: 5-ACAGCCAAGCTTGCATGC-3, to verify the successful insertion, thereby obtaining a PYW05 strain. According to the method described in Example 7, the yield of the obtained PYLW05 strain was determined to be 6.5 mg g⁻¹ DCW.

### Example 5: Knockdown of acetate kinase gene

Based on an acetate kinase gene sequence in the genome of *Synechococcus elongatus* PCC 7942, antisense RNA of 24bp was designed. A full-length sRNA sequence for acetate kinase (as set forth in SEQ ID NO. 20) was then artificially synthesized based on the backbone Mice and Trbcl sequences of sRNA.

Primers, i.e., SEQ ID NO. 15: 5-GGATCCTATCAATAAGTATTAGGTATATGG-3 and SEQ ID NO. 16: 5-GAATTCGCTGTCGAAGTTGAACAT-3, were designed to amplify the full-length sRNA sequence. The full-length sRNA sequence and the recombinant plasmid *pBA-acsA* obtained in Example 4 were double enzymatically cleaved with *Bam*HI and *EcoR*I*,* respectively, purified, and then ligated overnight at 16°C using T₄ DNA ligase. The ligation product was chemically transformed into *E. coli* competent cells. The transformed mixture was spread on an LB plate with kanamycin (50 µg/mL). The plasmid was extracted. Double enzymatic cleavage was performed to verify the constructed recombinant plasmid named pBA-*acsA-as-ackA.* Sequencing was performed by Shanghai Parsenor Biotechnology Co., Ltd.

The target gene was integrated into the natural site III in the chromosome of *Synechococcus elongatus* PCC 7942 via the extracted recombinant plasmid DNA by natural transformation based on the principle of homologous recombination to obtain a PYLW06 strain. According to the method described in Example 7, the yield of the obtained PYLW06 strain was determined to be 9.8 mg g⁻¹ DCW.

### Example 6: Further knockdown of acetyl-CoA carboxylase gene, ketosynthase gene and beta-ketoacyl-ACP synthase III gene

Based on the sequences of acetyl-CoA carboxylase gene, ketosynthase gene and beta-ketoacyl-ACP synthase III gene in the genome of *Synechococcus elongatus* PCC 7942, antisense RNA of 24bp was designed. A full-length sRNA sequence (as set forth in SEQ ID NO. 21) for acetyl-CoA carboxylase gene, ketosynthase gene and beta-ketoacyl-ACP synthase III was artificially synthesized based on the backbone Mice and Trbcl sequences of sRNA.

Primers, i.e., SEQ ID NO. 17: 5-GAATTCTATCAATAAGTATTAGGTATATGG-3 and SEQ ID NO. 18: 5- GAGCTCGCTGTCGAAGTTGAACAT-3, were designed to amplify the full-length sRNA sequence. The full-length sRNA sequence and the recombinant plasmid pBA*-acsA-as-ackA* obtained in Example 5 were double enzymatically cleaved with *EcoR*I and *Sac*I, respectively, purified, and then ligated overnight at 16°C using T₄ DNA ligase. The ligation product was chemically transformed into *E. coli* competent cells. The transformed mixture was spread on an LB plate with kanamycin (50 µg/mL). The plasmid was extracted. Double enzymatic cleavage was performed to verify the constructed recombinant plasmid named *pBA-acsA-as-ackA-as-accC-as-fabF-as-fabH.* Sequencing was performed by Shanghai Parsenor Biotechnology Co., Ltd.

The target gene was integrated into the natural site III in the chromosome of *Synechococcus elongatus* PCC 7942 via the extracted recombinant plasmid DNA by natural transformation based on the principle of homologous recombination to obtain a PYLW07 strain. According to the method described in Example 7, the yield of the obtained PYLW07 strain was determined to be 15.0 mg g⁻¹ DCW.

### Example 7: Production of PLA using recombinant cyanobacteria cells

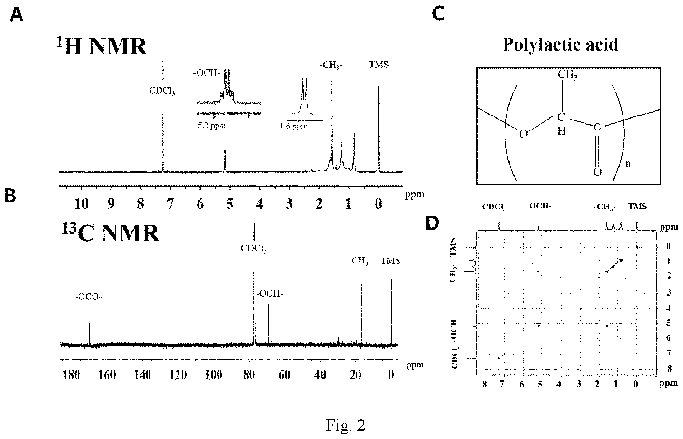

The recombinant cyanobacteria cell strain constructed in Example 1 was cultured in a BG-11 liquid culture medium at 30°C, with a ventilation rate of 5% (v:v) of carbon dioxide and at light intensity of 125 µmol photons m⁻² s⁻¹, until the OD₇₃₀ₙₘ reached 0.5. Then, 1 mol/L IPTG was added as an inducer for expression. The strain was centrifuged and collected when the growth OD reached the maximum. The recycled strain was freeze-dried, and a polymer substance accumulated within the strain was recycled with chloroform. The recycled polymer was subjected to a nuclear magnetic resonance (NMR) analysis. The results confirmed that the obtained polymer substance was PLA, as illustrated in Fig. 2.

For a quantitative experiment on PLA production by the strain, the yield of PLA was calculated by measuring its monomer content using gas chromatography-mass spectrometry (GC-MS), as disclosed in *"*Jung et al., Biotechnology & Bioengineering, 2010, 105 (1): 161-171".

### Example 8: Investigation on the influence of different concentrations of CO₂ on the growth of cyanobacteria

Cyanobacteria are bacteria that use CO₂ as a carbon source for growth. Therefore, the concentration of CO₂ introduced has a significant influence on cyanobacteria. In order to develop a high density fermentation strategy for cyanobacteria, the influence, on the growth of cyanobacteria, of different concentrations of CO₂ introduced into the culture medium during culture (other culture conditions were the same as in Example 7, using the PYLW07 strain) was first investigated in the present disclosure. When the volume ratio of CO₂ introduced to air was 1%, 2%, 5%, 7%, and 10%, the OD for 5-day growth was 2.5, 3.0, 3.5, 3.1, and 2.8, respectively. It was found that the growth OD₇₃₀ₙₘ of the *Synechococcus elongatus* PCC 7942 strain was up to 3.5 when the volume ratio of CO₂ to air was 5%.

### Example 9: Investigation on the influence of light intensity on the growth of cyanobacteria

Based on the conditions of Example 8, the influence of light intensities on the growth of cyanobacteria (under the optimal conditions of Example 8, other culture conditions were the same as in Example 7, using the PYLW07 strain) was further investigated in the present disclosure. According to the present disclosure, light intensities ranging from 125 to 1000 µmol photons m⁻² s⁻¹ were employed. It was found that the growth OD of cyanobacteria reached the maximum when the light increased to 500 µmol photons m⁻² s⁻¹, and the growth rate decreased when the light intensity exceeded 500 µmol photons m⁻² s⁻¹. When the light intensity reached 1000 µmol photons m⁻² s⁻¹, the cyanobacteria no longer grow under the stress of strong light. When the light intensity (in the unit of µmol photons m⁻² s⁻¹) was 125, 250, 500, and 750, the OD for 5-day growth was 2.0, 2.5, 3.5, and 2.2, respectively. However, cyanobacteria should not be subjected to excessively strong light before OD₇₃₀ₙₘ=1.0. Therefore, the light intensity of 125 µmol photons m⁻² s⁻¹ was selected before OD₇₃₀ₙₘ=1. O. With the growth, the light intensity was gradually increased to 500 µmol photons m⁻² s⁻¹, enabling the cyanobacteria to grow best, and the OD₇₃₀ₙₘ could reach 3.5 within 5 days.

### Example 10: Investigation on the influence of culture medium on the growth of cyanobacteria

As the optimal light intensity for the growth of cyanobacteria has been found in Example 9, it was further investigated whether the culture medium could be optimized to further increase the growth OD (under the optimal conditions of Example 9, other culture conditions were the same as in Example 7, using the PYLW07 strain). According to the present disclosure, BG-11 was used as a starting culture medium. Based on the formula of 5x BG-11 culture medium, the following ingredients were added to the BG-11 basal culture medium (L⁻¹): 1 g of Na₂CO₃, 5 g of MgSO₄·7H₂O, 5 g of NaNO₃, 2 mL of KH₂PO₄ (1 mol/L), and BG11 trace elements, BG11 trace element solution including 0.4 mM of H₃BO₃, 0.1 mM of MnCl_{2·}7H₂O, 0.004 mM of ZnSO₄·7H₂O, 0.01 mM of Na₂MoO₄·2H₂O, 0.003 mM of CuSO₄·5H₂O, and 0.001 mM of Co(NO3)₂·6H₂O. The culture medium was named as MBG-11, which can increase the growth compared to the previous one, with an increase in OD₇₃₀ₙₘ from 1.5 to 4.0.

Further, in order to investigate whether a high concentration of Na₂CO₃ leads to more biomass, the influence, on the growth of cyanobacteria, of different concentrations of Na₂CO₃ was investigated in the present disclosure, with Na₂CO₃ concentrations of 0.25 g/L, 0.50 g/L, 1.0 g/L, and 1.5 g/L. Through experiments, the OD of the 5-day growth was 3.5, 5.0, 7.0, and 3.0, respectively. When 1.0 g/L of Na₂CO₃ was added, the OD₇₃₀ₙₘ was up to 7.0.

### Example 11: High density fermentation of recombinant cyanobacteria PYLW07

Based on the experimental data in Example 7, the highest yield strain was determined to be the PLYW07 strain, which was used to investigate a high density fermentation strategy. Shake-flask fermentation was performed using the basal culture medium for the PCC 7942 strain (BG-11 liquid culture medium). For the high density culture strategy, the engineered PCC 7942 was cultured in a modified BG-11 solid culture medium (i.e., MBG-11 liquid culture medium) at 30°C using a self-made photobioreactor (as disclosed in Chinese Patent No: ZL 20222 0201535.5, which is incorporated herein by reference in its entirety) with an initial light intensity of 125 µmol photons m⁻² s⁻¹ for growth, and the light intensity was increased to 500 µmol photons m⁻² s⁻¹ after two days. For a culture with bubbling air containing CO₂ in a volume ratio of 5%, gas was bubbled through a vent valve with a 5 µm pore size at a flow rate of 50 mL min⁻¹. For PLA production, the PYLW07 strain was inoculated in 500 mL of MBG-11 liquid culture medium with 20 mg/L spectinomycin in an 800 mL flask, and CO₂-enriched air (5%, v/v) was continuously supplied. After the growth reached OD₇₃₀ₙₘ ranging from 0.4 to 0.6, cultures were induced by adding 1 mmol/L of IPTG and 2 mmol/L of theophylline.

The yield of PLA was detected by GC-MS. The final yield was 108 mg/L, equivalent to 23 mg/g DCW, approximately 270-fold that of the initial constructed strain under shake-flask culture. The molecular weight of PLA produced (weight-average molecular weight (Mw) of 62.5 kDa, number-average molecular weight (Mn) of 32.8 kDa) is at the highest level when compared with those reported in literatures. In addition, by using the high density culture strategy, the cell density was increased by 10-fold compared to the same engineering strain under shake-flask culture.

### Example 12: Collection of recombinant cyanobacteria cells

In order to conveniently and quickly collect cyanobacteria cells, the effect of natural sedimentation with a polyelectrolyte flocculant was explored in the present disclosure. A polyacrylamide flocculant was dissolved in water at room temperature at a ratio of 1: 300 (w/w) and at a ratio of 5: 100 (w/w), respectively. The dissolved polyacrylamide flocculant was then added to the fermentation liquor obtained by the high density culture at a ratio of 1.5% (v/v). The mixed culture was stirred for 2 min. The culture was then allowed to settle by gravity for 5 to 10 minutes. Flocculation efficiency was characterized by optical density measurements before and after sedimentation at an OD₇₃₀nm wavelength. The determined results suggest that the flocculation efficiency reached 90%

### Potential value in industry

A technical and economic bottleneck of the algal biomass industry is to harvest microalgae biomass on an industrial scale. The situation could be more complex when it comes microalgae due to the small cell size and the biomass concentration diluted in the culture. During the harvest, a huge amount of water is required to be removed, making such a process to be energy and cost intensive, which may account for approximately 30% of the total cost of biomass production. PLA is an inclusion of cells, and thus PLA particles can be collected together with the cells by centrifugation. When using a flocculant, the cells can be collected in a quick and convenient way, thereby having great potential industrial value.

The examples described above are provided to enable those skilled in the art to understand and apply the present disclosure. Various modifications to these examples will be readily apparent to those skilled in the art, and the generic principles defined herein may be applied to other examples without any inventive effort. Therefore, the present disclosure is not limited to the above examples. Modifications and variations made by those skilled in the art based on the principles of the present disclosure and without departing from the scope of the present disclosure should fall within the scope of the present disclosure.

## Claims

1. A genetically engineered strain of *Synechococcus elongatus,* wherein the genome of the genetically engineered strain is integrated with a coding sequence of exogenous D-lactate dehydrogenase gene, a coding sequence of exogenous propionyl-CoA transferase gene, and a coding sequence of exogenous polyhydroxyalkanoate synthase gene, enabling the genetically engineered strain to express exogenous D-lactate dehydrogenase, exogenous propionyl-CoA transferase, and exogenous polyhydroxyalkanoate synthase.

2. The genetically engineered strain according to claim 1, wherein the coding sequence of D-lactate dehydrogenase gene is under the control of a P_{trc} promoter, a P_{psba} promoter, or a P_{cpc560} promoter.

3. The genetically engineered strain according to claim 1, wherein the coding sequence of propionyl-CoA transferase gene is under the control of a P_{trc} promoter, a P_{psba} promoter, or a P_{cpc560} promoter.

4. The genetically engineered strain according to claim 1, wherein the coding sequence of polyhydroxyalkanoate synthase gene is under the control of a P_{trc} promoter, a P_{psba} promoter or a P_{cpc560} promoter.

5. The genetically engineered strain according to claim 1, wherein:
the coding sequence of exogenous D-lactate dehydrogenase gene is derived from *Lactobacillus bulgaricus:*
the coding sequence of exogenous propionyl-CoA transferase gene is derived from *Clostridium propionicum;* and
the coding sequence of exogenous polyhydroxyalkanoate synthase gene is derived from *Pseudomonas.*

6. The genetically engineered strain according to claim 1, wherein the genome of the genetically engineered strain is integrated with a coding sequence of exogenous acetyl-CoA synthase gene, enabling the genetically engineered strain to express the exogenous acetyl-CoA synthase gene.

7. The genetically engineered strain according to claim 1, wherein expression of acetate kinase gene in the genetically engineered strain is knocked down.

8. The genetically engineered strain according to claim 7, wherein the genome of the genetically engineered strain is integrated with a coding sequence of exogenous sRNA capable of inhibiting the expression of acetate kinase gene.

9. The genetically engineered strain according to claim 1, wherein expression of acetyl-CoA carboxylase gene, expression of ketosynthase gene, and/or expression of beta-ketoacyl-ACP synthase III gene in the genetically engineered strain are knocked down.

10. The genetically engineered strain according to claim 9, wherein the genome of the genetically engineered strain is integrated with a coding sequence of exogenous sRNA capable of inhibiting the expression of acetyl-CoA carboxylase gene, the expression of ketosynthase gene, and/or the expression of beta-ketoacyl-ACP synthase III gene.

11. The genetically engineered strain according to any one of claims 1 to 10, wherein the genetically engineered strain is a *Synechococcus elongatus* PCC 7942 strain.

12. A method for producing polylactic acid using carbon dioxide, the mehtod comprising:
1) providing the genetically engineered strain of *Synechococcus elongatus* according to any one of claims 1 to 11;
2) introducing carbon dioxide and culturing the genetically engineered strain under light; and
3) when a growth OD of the genetically engineered strain reaches the maximum, collecting and drying the genetically engineered strain, and recycling the polylactic acid in the strain.

13. The method according to claim 12, wherein in step 2), the carbon dioxide is introduced with a ventilation rate of 1.5 vvm, and a volume ratio of introduced carbon dioxide to air is 1: (1 to 10).

14. The method according to claim 12, wherein in step 2), an intensity of the light is from 125 µmol photons m⁻² s⁻¹ to less than 1000 µmol photons m⁻² s⁻¹, and preferably, the intensity of the light ranges from 125 µmol photons m⁻² s⁻¹ to 500 µmol photons m⁻² s⁻¹.

15. The method according to claim 12, wherein in step 2), the genetically engineered strain is cultured in a culture medium comprising sodium carbonate, sodium nitrate, dipotassium hydrogen phosphate, magnesium sulfate, calcium chloride, citric acid, ferric ammonium citrate, ethylenediamine tetraacetic acid, boric acid, manganese chloride, zinc sulfate, sodium molybdate, copper sulfate, and cobalt nitrate.

16. The method according to claim 15, wherein Na₂CO₃, MgSO₄·7H₂O, NaNO₃, KH₂PO₄, and trace elements are further added to the formula of the culture medium, and preferably, the trace elements are selected from H₃BO₃, MnCl₂7H₂O, ZnSO₄·7H₂O, Na₂MoO₄·2H₂O, CuSO₄·5H₂O, and Co(NO₃)₂·6H₂O.

17. The method according to claims 15 or 16, wherein a final concentration of the sodium carbonate in the culture medium ranges from 0.25 g/L to 1.5 g/L.

18. The method according to claim 12, wherein in step 2), the genetically engineered strain is cultured in the presence of an inducer, and preferably, the inducer is selected from one or more of IPTG and theophylline.

19. The method according to claim 12, wherein in step 3), said collecting the genetically engineered strain comprises:
mixing a polyelectrolyte flocculant with a culture solution containing the genetically engineered strain for 5 minutes, standing for 5 minutes, discarding a supernatant, and collecting a precipitate, and
preferably, the polyelectrolyte flocculant is a polyacrylamide flocculant.

20. The method according to claim 12, wherein in step 3), said recycling the polylactic acid in the strain is performed by using an organic solvent recycling or an inorganic aqueous phase extraction method, and
preferably, the organic solvent is selected from chloroform, 1,4-epoxydioxane, methyl-tetrahydrofuran, ethylene carbonate, acetone, and ethyl acetate.
